# FASCICULE DE BREVET EUROPEEN

(11) **EP 2 505 192 B1**
(45) Date de publication et mention de la délivrance du brevet: **04.05.2016**
(21) Numéro de dépôt: 12174311.6
(22) Date de dépôt: 12.09.2008
(51) Int. Cl.: A61K 9/06, A61K 47/10, A61K 47/38

(54) **Utilisation d'excipients en tant que conservateurs et composition pharmaceutique les comprenant**
Verwendung von Trägern als Konservierungsstoffe und pharmazeutische Zusammensetzung damit
Use of carriers as preservatives and pharmaceutical composition containing same

(30) Priorité: 12.09.2007 US 960043 P
(43) Date de publication de la demande: 03.10.2012
(62) Demande divisionnaire de: 08836906.1
(73) Titulaire: Galderma Research & Development, 06410 Biot (FR)
(72) Inventeur: Maitre, Lydie, 06100 Nice (FR)
(74) Mandataire: Chajmowicz, Marion

(56) Documents cités:
- EP-A- 1 803 464
- EP-A- 1 813 263
- WO-A-2006/075123
- US-A1- 2003 054 020
- US-A1- 2003 199 477

## Description

La présente demande se rapporte à l'utilisation d'une combinaison d'excipient au sein d'une composition pharmaceutique pour leur activité de conservateur et à la composition pharmaceutique topique la comprenant. La présente invention se rapporte à la combinaison d'excipient présentant une bonne activité de conservateur.

Une composition pharmaceutique ou cosmétique topique est souvent un bon substrat pour la prolifération des micro-organismes.
Il existe deux grandes familles de microorganismes, les bactéries et les fongiques. Les pharmacopées européenne et américaine, que l'homme de l'art dans le domaine plus particulier des produits pharmaceutiques se doit de suivre, ont sélectionné au total cinq germes représentatifs de ces familles, trois espèces de bactéries et deux sortes de fongiques.

Les trois espèces de bactéries suivantes sont représentatives de la population microbienne fréquemment rencontrée lors des contaminations et sont susceptibles de provoquer une infection chez l'homme. *L'Escherichia Coli* est une bactérie gram négative. Il a une paroi fine perméable aux petites molécules. Il a une forme de bâtonnet, dit bacille. Cette bactérie est présente essentiellement dans les déjections fécales. Sa présence peut provoquer diverses maladies telles que la gastro-entérite. Le *Staphylococcus aureus* est une bactérie gram positive. Il a donc une paroi plus épaisse qui est imperméable aux petites molécules. Sa forme est sphérique, il est dit coque, comme son nom l'indique. Le *Staphylococcus aureus* est l'espèce la plus pathogène du genre *Staphylococcus.* Il est responsable d'intoxications alimentaires, d'infections localisées suppurées Le *Pseudomonas aeruginosa,* est une bactérie gram négative du genre Pseudomonas. Les bacilles sont fins, droits et très mobiles grâce à un flagelle polaire. Il est pathogène et fréquemment rencontré dans les infections nosocomiales.

Les fongiques sont eucaryotes avec un noyau entouré d'une membrane et contenant les chromosomes. Il en existe deux sortes, les levures et les moisissures. Le *Candida Albicans* est l'espèce de levure la plus importante et la plus connue du genre *Candida.* C'est un organisme vivant à l'état naturel dans la bouche et le tube digestif de l'être humain. On le retrouve chez 80% de la population, et il n'entraine habituellement aucune maladie ou symptôme en particulier. C'est un organisme commensal saprophyte qui devient pathogène si l'organisme porteur s'affaiblit. La moisissure étudiée est l'*Aspergillus Niger.* Il est largement répandu (fruits et légumes moisis, fourrage, produits laitiers). Il est très utilisé dans l'industrie agroalimentaire pour la production de divers acides. Cette espèce peut être pathogène (aspergillose du conduit auditif, production d'ochratoxine).

Les sources possibles de contaminations microbiennes des produits topiques sont très nombreuses. Les micro-organismes proviennent principalement de l'homme ou de l'environnement et interviennent à différents moments du process, du stockage ou de l'utilisation du produit.

Le formulateur de composition pharmaceutique doit donc intégrer cette problématique très tôt dans le développement d'une composition topique. L'ajout d'agent conservateur est souvent la solution adoptée.

Un conservateur antimicrobien est une substance ou un ensemble de substances qui est ajouté volontairement à une formule afin d'éviter la prolifération des microorganismes. Ces conservateurs doivent être introduits en faible quantité, avoir un spectre d'action large, être dénué de toxicité et ne doivent pas palier à l'efficacité des principes actifs utilisés.
Un agent conservateur ne possède pas toujours un spectre d'activité assez large pour inhiber l'ensemble des germes énoncés précédemment. C'est pourquoi, leur association suivant leur activité est une voie souvent utilisé. En les combinant correctement, le système conservateur obtenu agit sur la totalité des microorganismes.

Il existe une grande variété d'agents conservateurs antimicrobiens utilisable dans les produits topiques. Cependant, si cette liste est généreuse pour les produits à usage cosmétique, elle est restreinte pour les produits pharmaceutiques. En effet, seulement une dizaine de conservateurs est usuellement utilisée dans ce milieu.
Cette différence vient du fait qu'il est imposé, lors de l'étude clinique, aux produits pharmaceutique, une parfaite tolérance ou innocuité sur l'homme.

Les conservateurs usuellement utilisés dans le domaine pharmaceutique sont, par exemple, les générateurs de formaldéhylde, tel l'imidazolidinylurée, ou la diazolidinylurée, les dérivés thiazinique tel le Kathon CG, les dérivés chlorés, tel la chlorhexidine, les dérivés phénoliques, tel les parabens, les dérivés alcooliques, tel le bronopol, le phénoxyéthanol, le bronopol, le chloroxylenol, les dérivés acides, tel l'acide sorbique, l'acide benzoîque ou le chlorure de benzalkonium.

Les conservateurs préférés et plus classiquement utilisés dans les compositions pharmaceutiques sont les parabens, notamment les méthylparaben, propylparaben, ethyparaben, butylparaben et le phénoxyéthanol seul ou en mélanges.

Les agents conservateurs les plus fréquents sont les parabens, idéalement associés au phénoxyéthanol.
Cependant, le phénoxyéthanol peut devenir une substance toxique à forte dose pour l'utilisateur. Pour des personnes saines, les concentrations nécessaires à la toxicité sont élevées. Toutefois le phénoxyéthanol est un allergène reconnu. Son utilisation peut provoquer des plaques d'eczémas chez certaines personnes.
Par ailleurs, Les parabens présentent eux aussi une toxicité à forte dose.

De façon générale, l'homme de l'art sait que les conservateurs usuellement utilisés dans les formules topiques peuvent être potentiellement sensibilisants, irritants et/ou allergisants. Pour l'ensemble de ces raisons, il devient important de limiter leur utilisation voir de les éliminer complètement des formules. Or, si un système conservateur n'est pas ajouté, la protection antimicrobienne ne sera pas effective. C'est pourquoi, il est important de déterminer une autre approche pouvant assurer cette fonction.

Certains facteurs, tels la température du milieu, le pH, le teneur en eau influencent la colonisation par les micro-organismes et l'homme de l'art a étudié la possibilité de les modifier. La température du milieu n'a pas d'effet réellement bactéricide ou fongicide. Les bactéries se développent vers 32,5°C et les fongiques vers 22,5°C. Si une formule est conservée en dessus ou en dessous de ces températures par exemple au réfrigérateur à + 4°C, les germes se mettront en sommeil et ne se développeront pas. Si la température redevient favorable, les microorganismes pourront de nouveau croître. Le maintien à haute (>+75°C) ou faible (<+4°C) température présente de nombreuses contraintes que ce soit lors de la production comme de l'utilisation.

Si le pH de la composition est très acide ou très basique, le développement des germes sera limité. Toutefois, les moisissures sont tolérantes à une gamme de pH très large, ainsi leur développement sera malgré tout possible. L'utilisation d'une composition topique à fort ou faible pH a également ses limites. En effet, l'application de solution ou crème très acide ou basique sur la peau peu avoir des effets irritants.

La teneur en eau est également un point très important. En effet, la présence d'eau est indispensable à la prolifération des germes. Ainsi, si le milieu est complètement anhydre, leur prolifération sera inhibée. Cependant, l'homme de l'art dans le milieu pharmaceutique et cosmétique ne peut pas envisager uniquement des compositions anhydres très souvent moins agréables que des compositions de type émulsion ou gel. Par ailleurs, de nombreux principes actifs pharmaceutiques sont solubles dans l'eau et nécessitent donc la présence de celle-ci au sein des compositions. Sans avoir à éliminer l'eau, l'homme de l'art doit prendre en compte l'activité en eau au sein de la composition. L'activité de l'eau n'est pas exactement la quantité d'eau, mais la quantité d'eau libre dans un milieu. L'eau libre est l'eau qui ne sert pas à solvater les molécules introduites dans le milieu aqueux.
Cette eau libre est donc complètement accessible aux germes pour assurer leur prolifération. L'activité de l'eau (aw) est une valeur intrinsèque du milieu étudié, elle ne dépend d'aucun paramètre extérieur. L'aw varie de 1,00 pour de l'eau pure à 0,00 pour un milieu complètement anhydre.
Les besoins en eau de chacun des germes est différent. Les bactéries par exemple sont plus sensibles à sa présence que les moisissures.
Le tableau 1 ci-dessous représente l'activité de l'eau nécessaire à différents germes pour pouvoir se développer.

**Tableau 1 : Valeurs de l'activité de l'eau nécessaire à la croissance de germes**

| Microorganisme | Activité en eau |
|---|---|
| Moyenne des bactéries | 0,91 |
| *Pseudomonas Aeruginosa* | 0,97 |
| *Escherichia coli* | 0,95 |
| *Staphilococcus aureus* | 0,86 |
| Moyenne des levures | 0,86 |
| *Candida Albicans* | 0,88 |
| Moyenne des moisissures | 0,80 |
| *Aspergillus Niger* | 0,77 |

Ainsi, si l'aw est inférieur à 0,77, les germes énoncés dans ce tableau, n'auront pas la faculté de se développer.
Il devient donc important de connaître les moyens qui induisent une réduction de l'activité de l'eau.
Le premier et le plus évident est de réduire la quantité introduite d'eau à l'intérieur des formule. Le second consiste à déterminer des molécules qui auraient un pouvoir solvatant important. Ainsi, l'eau serait plus mobilisée pour solvater ces molécules et serait moins disponibles pour les germes. Les humectant, les sels minéraux, les hydrocolloïdes peuvent jouer ce rôle.
L'utilisation de la mesure de l'activité en eau des formulations a fait l'objet très récemment d'un chapitre des méthodes générales de la pharmacopée américaine (« Application of water activity détermination to nonsterile pharmaceutical products <1112> USPC Official 5/1/07-7/31/07, 2007»).

La littérature décrit des compositions auto-protégées et listent des excipients connus pour présenter une activité de conservateur. Ainsi, Jon J.Kabara (et D.S. Orth dans "Preservative-free and self-preserving Cosmetics and Drugs - Principles and Practice ", edition Dekker, New York, 1996) liste différentes catégories d'excipients susceptibles d'avoir une activité de type conservateur au sein de la composition, tels, l'alcool, les tensioactifs, les esters ou acides gras, les phospholipides, antioxydants, agents chélatants. Cependant, bon nombre de ces ingrédients pris seul ou en mélange ne remplissent pas les critères exigées par les pharmacopées, ou les satisfont à des concentrations non acceptables au sein d'une composition pharmaceutique. Ils ne peuvent donc pas être utilisés tel que et facilement par l'homme de l'art confronté aux exigences réglementaires, au sein des compositions pharmaceutiques.

Les tensioactifs les plus actifs sont les cationiques. Cependant, ces derniers sont essentiellement utilisables au sein de compositions sous forme de shampooings, forme pharmaceutique non majeure dans l'activité. En effet, l'homme de l'art formule plus particulièrement des émulsions ou des gels.

Par ailleurs, J.J. Kabara a décrit que la prolifération des micro-organismes peut être empêchée avec de l'éthanol, mais à partir de 25% (v/v). Cependant, d'une part ces pourcentages sont extrêmement élevés pour une bonne tolérance d'une composition pharmaceutique topique, d'autre part, rien dans la littérature ne confirme les réductions effectivement observées de chaque micro-organisme telles qu'elles doivent l'être en accord avec les pharmacopées.

Le document WO 2006/075123 décrit des formulations comprenant 10 % d'éthanol, 5 % de propylène glycol, 0,01 % de palmitate d'ascorbyle, 53,3 % de glycerol dioléate et 28,7 % de phosphatidyl choline.

Le problème que se propose de résoudre ici la présente invention, est donc de trouver une alternative aux conservateurs classiques afin de protéger efficacement une composition topique, pharmaceutique, contre les microorganismes cités précédemment.

Par conservateurs connus, on entend, par exemple, les générateur de formaldéhylde, tel l'imidazolidinylurée, ou la diazolidinylurée, les dérivés thiazinique tel le Kathon CG, les dérivés chlorés, tel la chlorhexidine, les dérivés phénoliques, tel les parabens, les dérivés alcooliques, tel le bronopol, le phénoxyéthanol, le bronopol, le chloroxylenol, les dérivés acides, tel l'acide sorbique, l'acide benzoîque ou le chlorure de benzalkonium.

Les conservateurs les plus classiquement utilisés dans les compositions, notamment par la demanderesse sont les parabens, notamment les méthylparaben, propylparaben, ethyparaben butyl paraben et le phénoxyéthanol seul ou en mélanges.

Le problème que se propose de résoudre l'invention est donc de limiter, voire de supprimer les conservateurs dans une composition topique pharmaceutique.

Un problème particulier que doit résoudre l'invention est de protéger efficacement une composition topique pharmaceutique tout en répondant aux critères exigés par les pharmacopées européennes et américaines. En effet, la composition pharmaceutique ayant pour finalité de devenir un produit pharmaceutique avec une autorisation de mise sur le marché, il est impératif que la composition selon l'invention satisfasse à ces critères. Afin de vérifier ce point et déterminer l'action antimicrobienne des différents mélanges d'excipient selon l'invention, il existe un protocole de test appelé le PET (preservative efficacity test). Le PET consiste à inoculer artificiellement les compositions par un nombre connu de germes, puis à déterminer la décroissance de ces derniers dans les compositions testées à des temps donnés. Le protocole de ce test est mis au point afin de respecter les recommandations des pharmacopées européenne et américaine. Le test ainsi que les recommandations des pharmacopées européenne et américaine sont décrites à l'exemple 1 de la présente demande. Le mélange d'excipients comme conservateurs décrit dans la présente invention doit donc remplir les critères de ces pharmacopées dans le PET.

La portée de la présente invention est définie par les revendications. Les éléments décrits dans la description en dehors de cette portée sont fournis pour information seulement.

La présente description décrit une composition pharmaceutique topique contenant un mélange d'excipients comme conservateur, caractérisé en ce que la composition ne contient pas d'autre conservateur classique.

Par composition pharmaceutique topique, on entend toute composition que l'homme de l'art peut envisager et notamment mais non exclusivement, les compositions sous forme liquide, ou pâteuse, et plus particulièrement sous forme d'émulsions, de crèmes, de laits, de pommades, de tampons imbibés, de syndets, de lingettes, de gels, de sprays, de mousses, de lotions, de sticks, de shampoings, ou de bases lavantes.

En particulier, parce que la protection des compositions contenant de l'eau est la plus complexe, la demande décrit une composition pharmaceutique topique contenant au moins une phase aqueuse contenant de l'eau, soit préférentiellement, une émulsion ou une lotion.

La composition pharmaceutique comprenant le mélange d'excipient conservateur décrit dans la demande est destinée au traitement de la peau et est donc administrée par voie topique. Par voie topique, on entend une application sur la peau ou les muqueuses.

Par mélange d'excipient conservateur, on entend un mélange d'ingrédient classiquement utilisé comme base formulaire, au sein de laquelle sont introduits le ou les actifs. On peut lister à titre indicatif d'excipients, les solvants, notamment de type éthanolique comme l'éthanol, les tensioactifs, les humectants, les glycols.

Comme indiqué précédemment, l'homme de l'art sait que l'éthanol présente une action antimicrobienne et antifongique importante à partir de 25% au sein de la composition. Mais cette concentration est difficilement compatible avec une bonne tolérance pour une application topique sur la peau d'un patient.

La demanderesse a découvert de manière surprenante qu'en associant l'éthanol avec d'autres composés excipients, il était possible d'obtenir une bonne protection anti-microbienne tout en réduisant la concentration en éthanol au sein de la composition.

Les excipients préférés de la présente divulgation à associer à l'éthanol sont notamment les humectants, les anti-oxydants, les tensioactifs, les chélatants, et/ou les épaississants.

A titre d'humectant on entend les humectants utilisés fréquemment en voie topique. Leur action permet de maintenir le taux d'humidité dans le stratum corneum. Les humectants utilisables selon la présente divulgation sont notamment mais non exclusivement les glycérol et dérivés, les glycols, tel le propylène glycol, les polyéthylènes glycol.
Les humectants préférés selon la présente divulgation sont le propylène glycol, la glycérine ou glycérol, ou le polyéthylène glycol

A titre d'anti-oxydants, on entend selon la présente divulgation les anti-oxydants de type naturels, tels les ascorbiques, les citriques, les tartriques, les lactates, les tocophérols, ou les anti-oxydants synthétiques, tel le butylhydroxyanisole (BHA), le butylhydroxytoluène (BHT), le butylhydroquinone tertiaire (TBHQ), ou le propylgallate.

A titre de tensioactifs utilisables selon la présente divulgation, on peut citer, les tensioactifs anioniques, cationiques, amphotères ou non-ioniques. Les tensioactifs cationiques sont préférés et notamment les ammoniums quaternaires et les aminimides.

A titre d'agents chélatants utilisables selon la présente divulgation, on peut nommer les chélatants faibles ou labiles et les chélatants forts tel l'EDTA.

A titre d'agent épaississants utilisables selon la présente divulgation, on entend notamment les dérivés d'acide acrylique, tels les Carbopol (tel Ultrez 10 NF), les dérivés cellulosiques, tel le Natrosol, et les gomme xanthane.

Selon la présente divulgation, le mélange d'excipient présentant une bonne activité antimicrobienne comprend au sein de la composition pharmaceutique, au moins:
- De l'éthanol,
- Au moins un humectant, un anti-oxydants, un tensioactifs, un chélatants, et/ou un épaississant.

La demande décrit plus particulièrement une composition pharmaceutique topique comprenant un mélange d'excipients contenant au moins de l'éthanol et des humectants.

Les humectants préférés selon la présente divulgation sont à titre indicatif, le propylène glycol, la glycérine ou le polyéthylène glycol, notamment le PEG 400 utilisés seuls ou en mélange.

Selon la présente divulgation, le mélange d'excipient présentant une bonne activité antimicrobienne qui comprend, au sein de la composition pharmaceutique, au moins:
- De l'éthanol,
- Au moins un humectant, choisi parmi le propylène glycol, la glycérine ou glycérol, le polyéthylène glycol, seul ou en mélange.

En particulier, la demande décrit un mélange d'excipient présentant une bonne activité antimicrobienne qui comprend au sein de la composition pharmaceutique, au moins:
- entre 5 et 25% d'éthanol,
- entre 1 et 50 % d'un humectant, choisi parmi le propylène glycol, la glycérine ou glycérol, le polyéthylène glycol, seul ou en mélange.
Tout particulièrement, la composition décrite dans la demande comprend également au sein du mélange d'excipient conservateur, un antioxydant.

Préférentiellement l'antioxydant est l'ascorbyl palmitate à une concentration d'au moins 0,5 %, de préférence à une concentration de 1% en poids par rapport au poids total de la composition.

Selon un autre mode de la présente divulgation, la composition comprend également au sein du mélange d'excipient conservateur, un épaississant.

Préférentiellement l'épaississant est un dérivé cellulosique, tel le Natrosol, présent à une concentration d'au moins 0,5 %, de préférence à une concentration de 1,5% en poids par rapport au poids total de la composition.

La présente divulgation décrit également la composition pharmaceutique comprenant le mélange d'excipients selon l'invention.

La présente demande décrit aussi une composition pharmaceutique topique caractérisée en ce qu'elle comprend un mélange d'excipients présentant une activité anti-microbienne, le dit mélange d'excipients comprenant :
- au moins un excipient choisi parmi l'éthanol et au moins un excipients choisis parmi les humectant(s), les anti-oxydant(s), les tensioactif(s), les chélatant(s), et les épaississant(s).
- un rapport éthanol / phase aqueuse allant de 10 à 15 %.

Selon un mode particulier décrit dans la demande, la composition comprend donc un mélange d'excipient comme conservateur, comprenant :
- Un antioxydant,
- Un humectant,
- Un rapport éthanol / phase aqueuse allant de 10 à 15 %.

Selon un autre mode particulier décrit dans la demande, la composition selon la présente divulgation peut aussi prendre en compte l'activité en eau au sein de la composition.
Dans un mode préféré de la présente divulgation, la composition comprend donc un mélange d'excipient comme conservateur, comprenant :
- l'ascorbyl palmitate à 1% en masse
- le propylène glycol à 5% en masse
- Un pourcentage en eau inférieur à 50% en masse
- Un rapport éthanol / phase aqueuse allant de 10 à 15 %.

La présente divulgation porte également sur une composition pharmaceutique topique, caractérisée en ce qu'elle comprend un mélange d'excipients présentant une activité anti-microbienne, le dit mélange d'excipients comprenant:
- au moins un excipient choisi parmi l'éthanol et au moins un excipient choisi parmi les humectant(s), les anti-oxydant(s), les tensioactif(s), les chélatant(s), et les épaississant(s).
- un rapport éthanol / phase aqueuse allant de 10 à 15 %, et
De préférence, ladite composition pharmaceutique topique contient de 0 à 1,8% en masse de conservateurs choisis parmi les générateurs de formaldéhylde, tel l'imidazolidinylurée, ou la diazolidinylurée, les dérivés thiazinique tel le Kathon CG, les dérivés chlorés, tel la chlorhexidine, les dérivés phénoliques, tel les parabens, les dérivés alcooliques, tel le bronopol, le phénoxyéthanol, le bronopol, le chloroxylenol, les dérivés acides, tel l'acide sorbique, l'acide benzoïque et/ou le chlorure de benzalkonium.

Ladite composition est de préférence sous forme d'émulsion.

Ledit mélange d'excipients de la composition telle que définie plus haut est un un mélange d'éthanol, d'humectant(s), d'anti-oxydant(s), de tensioactif(s), de chélatan(s), et/ou d'épaississant(s).

Ledit mélange d'excipients comprend de préférence ;
- entre 5 et 25% d'éthanol,
- entre 1 et 50 % d'humectant(s) choisi(s) parmi le propylène glycol, la glycérine ou glycérol, et/ou le polyéthylène glycol.
De préférence l'humectant est le propylène glycol.
Ledit mélange d'excipients comprend également un antioxydant, qui est de préférence l'ascorbyl palmitate.
Ledit mélange d'excipients peut comprendre en outre un épaississant présent à une concentration d'au moins 0,5% en masse. Ledit épaississant est de préférence un dérivé cellulosique tel que le Natrosol.

Ladite composition telle que décrite plus haut comprend un rapport éthanol/phase aqueuse est compris de 10 à15%.

Ledit mélange d'excipients comprend plus particulièrement ;
- de l'ascorbyl palmitate à une concentration entre 0,5% et 1% en masse
- le propylène glycol à 5% en masse
- un pourcentage en eau inférieur à 50% en masse
- un rapport éthanol / phase aqueuse allant de 10 à 15 %.

Plus particulièrement la composition pharmaceutique topique telle que définie plus haut contient moins de 0,8% de parabens, et de préférence moins de 0,4% de propylparaben.

La composition pharmaceutique selon la présente divulgation est utilisable comme médicament.

En particulier, la présente divulgation porte également sur l'utilisation de la composition selon la présente divulgation pour la préparation d'un médicament destiné à traiter les affections dermatologiques, notamment humaines.

Il va être maintenant donné, à titre d'illustration et sans aucun caractère limitatif, diverses formulations de compositions selon l'invention.

### Exemple 1 : PET et exigences des pharmacopées européennes et américaines.

Ce test consiste à contaminer artificiellement des aliquotes de formulation au moyen d'inoculum calibré de microorganismes spécifiques et à leur incubation à des températures précis. Des prélèvements d'échantillons à partir des aliquotes contaminés sont effectués à intervalles de temps donnés afin de dénombrer les micro-organismes.

La pharmacopée européenne décrit des exigences qui doivent être respectées lors de ce test. Elle impose de tester les germes suivants :

### Les bactéries

*Pseudomonas aeruginosa,* Référence ATCC 9027 ; NCIMB 8626 ; CIP 82.118.
*Staphylococcus aureus,* Référence ATCC 6538 ; NCTC 10788 ; NCIMB 9518 ; CIP 4.83.

### Les fongiques

*Candida albicans,* Référence ATCC 10231 ; NCPF 3179 ; IP 48.72.
*Aspergillus Niger,* Référence ATCC 16404 ; IMI 149007 ; IP 1431.83. [34]

Elle impose également d'étudier les réductions de population des microorganismes introduits dans la formulation selon deux critères d'acceptabilités.
Les critères A étant les plus stricts, il est généralement demandé de les satisfaire.
Dans des cas justifiés, lorsque les critères A ne peuvent être respectés, par exemple en raison d'une augmentation du risque de réactions indésirables, les critères B, moins drastiques, s'appliquent.

**Tableau 2: Critères de la pharmacopée européenne**

| | **Réduction logarithmique au temps demandés** | | | | |
|---|---|---|---|---|---|
| | Critères | T48h | T7j | T14j | T28j |
| **Bactéries** | A | 2 | 3 | --- | NI |
| | B | --- | --- | 3 | NI |
| **Fongiques** | A | --- | --- | 2 | NI |
| | B | --- | --- | 1 | NI |

| | | | | | |
|---|---|---|---|---|---|
| NI (Non Increase) signifie qu'il ne doit pas avoir d'augmentation du nombre de germes. | | | | | |

La pharmacopée américaine décrit, elle aussi, des exigences qui doivent être respectées afin que la formulation soit protégée. Elle impose également de tester les germes suivant :

### Les bactéries

*Escherichia coli,* Référence ATCC 8739
*Pseudomonas aeruginosa,* Référence ATCC 9027.
*Staphylococcus aureus,* Référence ATCC 6538.

### Les fongiques

*Candida albicans,* Référence ATCC 10231.
*Aspergillus Niger,* Référence ATCC 16404.

Elle impose des réductions logarithmiques de la population de microorganismes qui sont différents de celle de la pharmacopée européenne.
Dans le cas des produits topiques, les conditions sont :

**Tableau 3: Critères de la pharmacopée américaine**

| **Réduction logarithmique au temps demandés** | | | | |
|---|---|---|---|---|
| | T48h | T7j | T14j | T28j |
| **Bactéries** | --- | --- | 2 | NI |
| **Fongiques** | --- | --- | NI | NI |

### Exemple 2 : Etude de l'action antimicrobienne de l'éthanol seul.

Cette étude est liée aux formes « lotions » très courantes en dermatologie. L'éthanol est un excipient fréquemment utilisé pour ce type de formule.
Le but de cette étude est de déterminer la proportion limite en éthanol qui assure la conservation antimicrobienne sans addition de conservateurs. L'effet d'excipients complémentaires a également été évalué dans le but de réduire la concentration d'éthanol inhibitrice.

Recherche du rapport éthanol/eau satisfaisant les critères des différentes pharmacopées en vigueur.
J.J. Kabara en 1997 a décrit que la prolifération des micro-organismes peut être empêchée à partir de 25% (v/v) d'éthanol.
Toutefois, il ne décrit pas dans cet extrait, les réductions observées de chaque microorganisme étudié lors du PET. Il ne nous est donc pas possible d'affirmer que les critères des pharmacopées européenne et américaine sont satisfaits à cette concentration.
Des mélanges simples éthanol/eau ont donc été étudiés dans ce sens.

Les concentrations en éthanol ont varié de 5% à 30% par pas de 5%.

### Résultats des analyses

**Tableau 4: Concentration d'éthanol 5%**

| | **Réduction observée en log** | **Critères à respecter** | **Satisfait, Non satisfait** | **Conclusion générale** |
|---|---|---|---|---|
| T2j | Sta : 0.2 | **Eur. Critère A** | Sta : Non satisfait | **Ne satisfait pas les critères A de la pharmacopée européenne.** |
| | Pseu : 0.4 | 2log de réduction pour les bactéries | Pseu : Non satisfait | |
| T14j | Coli : 0.2 | **Eur. Critère B** | Sta : Satisfait | **Ne satisfait pas les critères B de la pharmacopée européenne.** |
| | Sta : 5.8 | 3log de réduction pour les bactéries | Pseu : Non satisfait | |
| | Pseu : 0.9 | | | |
| | | **USP.** | Coli : Non satisfait | **Ne satisfait pas les critères de l'USP.** |
| | | 2log de réduction pour les bactéries | Sta : Satisfait | |
| | | | Pseu : Non satisfait | |

**Tableau 5: Concentration d'éthanol 10%**

| | **Réduction observée en log** | **Critères à respecter** | **Satisfait, non satisfait** | **Conclusion générale** |
|---|---|---|---|---|
| T2j | Sta : 1 | **Eur. Critère A** | Sta : Non satisfait | **Ne satisfait pas les critères A de la pharmacopée européenne.** |
| | Pseu : 1.6 | 2log de réduction pour les bactéries | Pseu : Non satisfait | |
| T14j | Coli : 0.9 | **Eur. Critère B** | Sta : Satisfait | **Ne satisfait pas les critères B de la pharmacopée européenne.** |
| | Sta : 5.8 | 3log de réduction pour les bactéries 1 log de réduction pour les fongiques | Pseu : Satisfait | |
| | Pseu : 6 | | Asp : Non satisfait | |
| | Asp : 0.2 | | | |
| | | **USP.** | Coli : Non satisfait | **Ne satisfait pas les critères de l**'**USP**. |
| | | 2log de réduction pour les bactéries pas de croissance pour les fongiques | Sta : Satisfait | |
| | | | Pseu : Satisfait | |
| | | | Asp : Satisfait | |

5

**Tableau 6: Concentration d'éthanol 15%**

| | Réduction observée en log | Critères à respecter | Satisfait, non satisfait | **Conclusion générale** |
|---|---|---|---|---|
| T2j | Sta : 4 | **Eur. Critère A** | Sta : Satisfait | **Ne satisfait pas les critères A de la pharmacopée européenne.** |
| | Pseu : 4 | 2log de réduction pour les bactéries | Pseu : Satisfait | |
| T14j | Coli : 6 | **Eur. Critère A** | Can : Satisfait | |
| | Sta : 6 | 2log de réduction pour les fongiques | Asp : Non satisfait | |
| | Pseu : 6 | | | |
| | Can : 3.9 | **Eur. Critère B** | Sta : Satisfait | **Ne satisfait pas les critères B de la pharmacopée européenne.** |
| | Asp : 0.6 | 3log de réduction pour les bactéries | Pseu : Satisfait | |
| | | | Can : Satisfait | |
| | | 1 log de réduction pour les fongiques | Asp : Non satisfait | |
| | | **USP.** | Coli : Satisfait | **Satisfait les critères de l'USP.** |
| | | 2log de réduction pour les bactéries pas de croissance pour les fongiques | Sta : Satisfait | |
| | | | Pseu : Satisfait | |
| | | | Asp : Satisfait | |

**Tableau 7: Concentration d'éthanol 20%**

| | **Réduction observée en log** | **Critères à respecter** | **Satisfait, non satisfait** | **Conclusion générale** |
|---|---|---|---|---|
| T2j | Sta : 4 | **Eur. Critère A** | Sta : Satisfait | **Ne satisfait pas les critères A de la pharmacopée européenne.** |
| | Pseu : 4 | 2log de réduction pour les bactéries | Pseu : Satisfait | |
| T14j | Coli : 6 | **Eur. Critère A** | Can : Satisfait | |
| | Sta : 6 | 2log de réduction pour les fongiques | Asp : Non satisfait | |
| | Pseu : 6 | | | |
| | Can : 3.9 | **Eur. Critère B** | Sta : Satisfait | **Satisfait les critères B de la pharmacopée européenne.** |
| | Asp : 1.6 | 3log de réduction pour les bactéries 1 log de réduction pour les fongiques | Pseu : Satisfait | |
| | | | Can : Satisfait | |
| | | | Asp : Satisfait | |
| | | **USP.** | Coli : Satisfait | **Satisfait les critères de l'USP.** |
| | | 2log de réduction pour les bactéries pas de croissance pour les fongiques | Sta : Satisfait | |
| | | | Pseu : Satisfait | |
| | | | Asp : Satisfait | |

**5 Tableau 8: Concentration d'éthanol 25%**

| | **Réduction observée en log** | **Critères à respecter** | **Satisfait, non satisfait** | **Conclusion générale** |
|---|---|---|---|---|
| T2j | Sta : 4 | **Eur. Critère A** | Sta : Satisfait | **Satisfait les critères A de la pharmacopée européenne.** |
| | Pseu : 4 | 2log de réduction pour les bactéries | Pseu : Satisfait | |
| T14j | Coli : 6 | **Eur. Critère A** | Can : Satisfait | |
| | Sta : 6 | 2log de réduction pour les fongiques | Asp : Satisfait | |
| | Pseu : 6 | | | |
| | Can : 3.9 | **Eur. Critère B** | Sta : Satisfait | **Satisfait les critères B de la pharmacopée européenne.** |
| | Asp : 3.8 | 3log de réduction pour les bactéries 1 log de réduction pour les fongiques | Pseu : Satisfait | |
| | | | Can : Satisfait | |
| | | | Asp : Satisfait | |
| | | **USP.** | Coli : Satisfait | **Satisfait les critères de l'USP.** |
| | | 2log de réduction pour les bactéries pas de croissance pour les fongiques | Sta : Satisfait | |
| | | | Pseu : Satisfait | |
| | | | Asp : Satisfait | |

Ces résultats montrent qu'à partir d'une concentration en éthanol de 15% dans le mélange aqueux, son action bactéricide satisfait les critères A de la pharmacopée européenne et les critères de l'USP. Toutefois, cette concentration est insuffisante pour inhiber complètement les fongiques. En augmentant la concentration d'éthanol à 25%, l'action fongicide est satisfaisante pour l'ensemble des pharmacopées. Mais la concentration à laquelle il présente une activité fongicide est difficilement compatible avec une bonne tolérance pour une application topique sur la peau d'un patient.

### Exemple 3 : Etude de l'action antimicrobienne de l'éthanol couplé à des humectants.

La demanderesse a testé le mélange d'éthanol avec des humectants afin de réduite la concentration d'éthanol au sein de la composition et d'en évaluer la protection anti-microbienne résultante.

Trois excipients utilisés comme humectants sont fréquemment utilisés en voie topique. Leur action permet de maintenir le taux d'humidité dans le stratum corneum. Le propylène glycol (PG), le glycérol et le PEG 400 sont des composés de ce type. L'impact de ces excipients sur les concentrations d'éthanol précédemment rapportées a été étudié.

Formules développée des humectants utilisées

| | | |
|---|---|---|
| 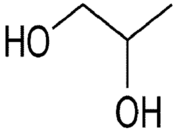 | 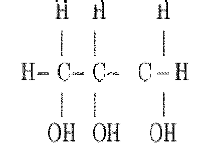 | 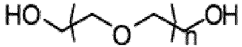 |
| Le propylène glycol | le glycérol | le polyéthylène glycol |

Un nombre limité de combinaisons entre les humectants et l'éthanol a été étudié.
A une concentration donnée en humectant, la plus faible concentration en éthanol qui satisfait les différents critères a été recherchée.
Chacun des trois humectants a été incorporés à différents concentrations variant de 0% à 30% par pas de 5% à des solutions en éthanol, variant dans les mêmes proportions. Ainsi 49 solutions été testés pour chaque humectant.

### Conclusion sur les actions antimicrobiennes des mélanges humectant/éthanol Propylène glycol :

10% de PG permettent de réduire la concentration d'éthanol de 25% à 15% pour satisfaire les critères A de la pharmacopée européenne.
30% massique dans une solution aqueuse permet de satisfaire les critères de l'USP sans ajout d'éthanol.
De plus à cette concentration, les bactéries sont inhibées en satisfaisant les critères A de la pharmacopée européenne.
Le PG a donc une action sur l'ensemble des bactéries (gram+ et gram- confondus).
Elle est toutefois limitée sur les fongiques. En effet, seul le PG n'assure pas la décroissance de ces germes, en particulier l'Aspergillus Niger. Il est donc nécessaire d'utiliser de l'éthanol pour éviter la prolifération de ces derniers

### Glycérine :

5% en masse de glycérine permettent de réduire la concentration en éthanol de 25% à 15% pour satisfaire les critères A de la pharmacopée européenne.

La zone d'acceptabilité globale des critères de la pharmacopée européenne est similaire à celle d'obtenue avec le PG.
10% en masse de glycérine permettent de réduire la concentration en éthanol de 15% à 5% pour satisfaire les critères de l'USP.

Toutefois, l'action bactéricide de la glycérine est moins importante que celle du PG. En effet, une solution aqueuse à 30% massique de glycérine permet de satisfaire uniquement les critères B de la pharmacopée européenne pour les bactéries, mais ne satisfait pas les critères de l'USP. L'élimination des fongiques, en particulier de l'*Aspergillus Niger,* est toujours un point bloquant qui nécessite l'apport important d'éthanol.

### Polyéthylène glycol 400 :

20% massique de PEG 400 permettent de réduire la concentration d'éthanol de 25%à 10% pour satisfaire les critères A de la pharmacopée européenne.
20% massique de PEG 400 permettent de réduire la concentration d'éthanol de 15% à 5% pour satisfaire les critères de l'USP.
L'action bactéricide est moins importante que celle du PG, mais supérieur à celui du glycérol.

Les humectants testés ont permis de réduire la concentration d'éthanol introduit au sein de la solution aqueuse.
Suivant les pharmacopées, les solutions suivantes de type « lotion » sont celles permettant d'assurer la protection antimicrobienne sans ajout de conservateurs. (Tableau n°9)

**Tableau 9: solutions assurant la protection antimicrobienne.**

| | **Pharma Europe** | **Pharma USP** |
|---|---|---|
| **Propylène Glycol (PG)** | à partir de 15% EtOH | à partir de 30% PG |
| | à partir de 5% de PG | à partir de 0% EtOH |
| **Glycérine** | à partir de 15% EtOH | à partir de 5% EtOH |
| | à partir de 5 % Glycérine | à partir de 10% Glycérine |
| **Polyéthylène glycol(PEG)** | à partir de 10% EtOH | à partir de 5% EtOH |
| | à partir de 20% PËG | à partir de 20% PEG |

Les humectants étudiés favorisent l'action antimicrobienne de l'éthanol tout en permettant de diminuer sa concentration dans la composition. Toutefois, leurs actions ne sont pas similaires. Il est possible de déterminer un ordre suivant leur action bactéricide.

### PG > PEG 400 > glycérine

Par la suite, il sera donc plus intéressant de sélectionner le PG ou le PEG comme humectant pour leurs propriétés sur les bactéries.

### Exemple 4 : Etude de l'activité de l'eau des compositions

L'action inhibitrice démontrée avec l'ajout d'éthanol et des trois humectants peut être interprétée comme bactéricide et fongicide directe.
Toutefois, ces excipients ont également la faculté de modifier la quantité d'eau libre disponible dans la formulation. Leur action peut donc être liée à l'activité de l'eau.

De plus, la viscosité des lotions à usage topique peut être potentiellement augmentée par l'addition d'épaississant (souvent des hydrocolloides). Ces derniers ont par définition la particularité de piéger les molécules d'eau libre présentes dans une formulation. Ils ont donc la propriété de réduire l'activité de l'eau, ce qui influence la prolifération microbienne.

L'étude consiste à mesurer l'influence des différentes compositions, lotions et gels et de relier l'activité en eau aux critères d'acceptation des différentes pharmacopées.

### Activité de l'eau des mélanges Glyco-Ethanoliques

### Voir la méthode d'analyse en annexe

Les humectants étudiés précédemment ont également été analysés à travers l'activité en eau.

### Effet du Propylène Glycol (PG)

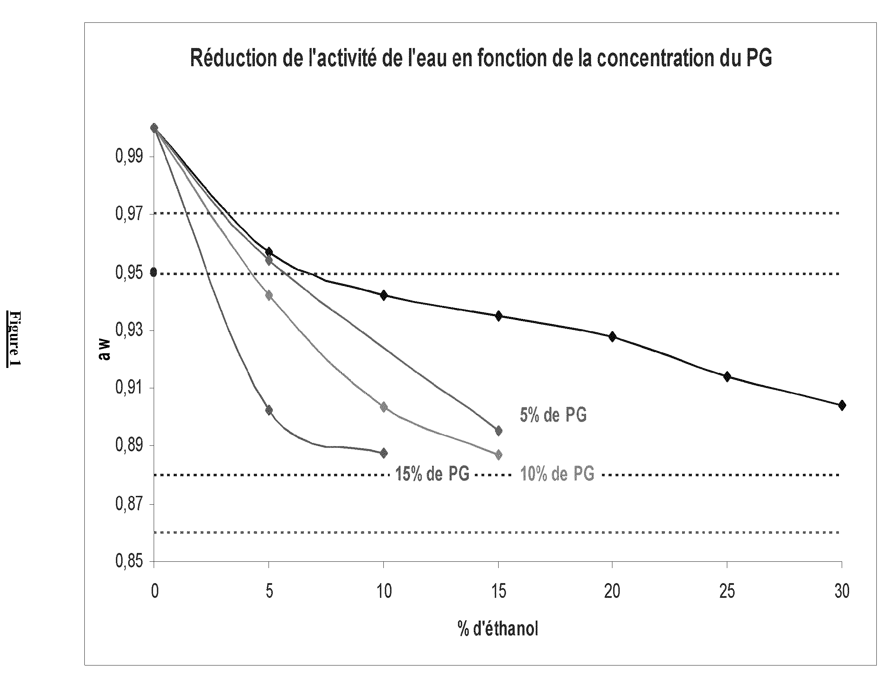

### Figure n°1 : Réduction de l'activité de l'eau en fonction de la concentration du PG

### Effet du glycérol

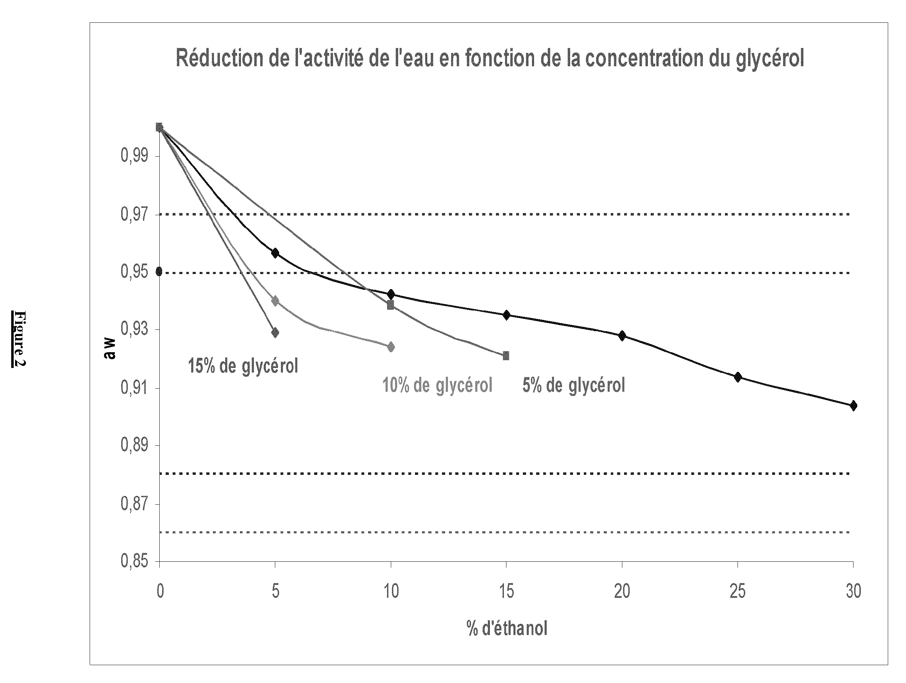

### Figure n°2 : Réduction de l'activité de l'eau en fonction de la concentration du glycérol

### Effet du PEG 400

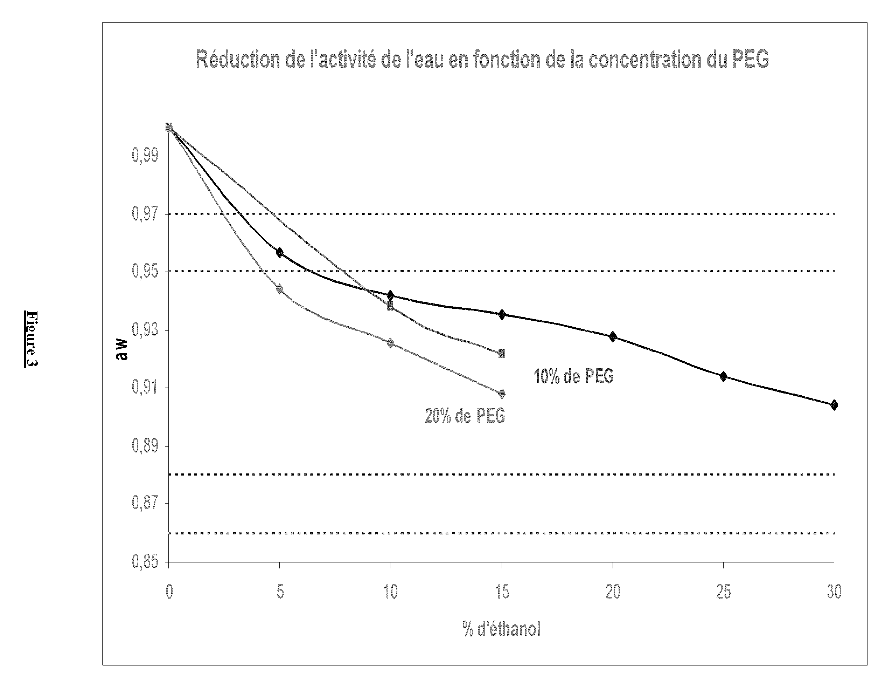

### Figure n°3 : réduction de l'activité de l'eau en fonction de la concentration du PEG

A concentration égale, le propylène glycol réduit l'activité de l'eau de manière plus importante que le PEG 400, la glycérine et que l'éthanol.

Exemple A : mélanges10% d'humectant /10% d'éthanol
A_{W} PG = 0.9036
A_{W} Glycérine : 0.9242
A_{W} PEG 400 : 0.9380

### PG > Glycérine > PEG

### Exemple B :

A_{W} 30% PG = 0,8906
A_{W} 30% éthanol = 0, 9040
A_{W} 30% glycérine = 0,9070

### PG > éthanol > glycérine

Il est donc plus intéressant de sélectionner le PG pour sa meilleure action sur l'activité de l'eau.

### Exemple 5 : Relation entre l'activité en eau des formules et la satisfaction des critères pharmacopée

| *Tableau n°10 : Relation entre les valeurs de a_{W} et l'action PET* | | | | | |
|---|---|---|---|---|---|
| | **Composition** | | **Aw** | **PET** | **Valeur limite de a_{W} pour assurer la croissance** |
| | | | | | a_{W} = 0.97 *Pseu* |
| | | 5% d'EtOH | **0,9564** | ni A ni B ni USP | a_{W} = 0.95 *Coli* |
| | 20% PEG 400 | 5% EtOH | **0,9442** | USP | |
| | | 10% d'EtOH | **0,9422** | Ni A ni B ni USP | |
| | 10% gly | 5% EtOH | **0,9400** | USP | |
| | | 15% d'EtOH | **0,9382** | USP | |
| | 5% gly | 10% EtOH | **0,9382** | USP | |
| | 10% PEG 400 | 10% EtOH | **0,9380** | USP | |
| | 15% gly | 5% EtOH | **0,9290** | USP | |
| | | 20% d'EtOH | **0,9280** | USP, EP B | |
| | 20% PEG 400 | 10% EtOH | **0,9258** | USP, EP A | |
| | 10% gly | 10% EtOH | **0,9242** | USP | |
| | 10% PEG 400 | 15% EtOH | **0,9218** | USP, EP A | |
| | 5% gly | 15% EtOH | **0,9210** | USP, EP A | |
| | | 25% d'EtOH | **0,9140** | USP, EP A | |
| | 20% PEG 400 | 15% EtOH | **0,9082** | USP, EP A | |
| | 30% gly | 0% EtOH | **0,9070** | Ni A ni B ni USP | |
| | | 30% d'EtOH | **0,9040** | USP, EP A | |
| | 10% PG | 10% EtOH | **0,9036** | USP | |
| | 15% PG | 5% EtOH | **0,9026** | USP | |
| | 5% PG | 15% EtOH | **0,8954** | USP, EP A | |
| | 30% PG | 0% EtOH | **0,8906** | USP | |
| | 15% PG | 10% EtOH | **0,8876** | USP | |
| | 10% PG | 15% EtOH | **0,8868** | USP, EP A | a_{W} = 0.88 *Can* |

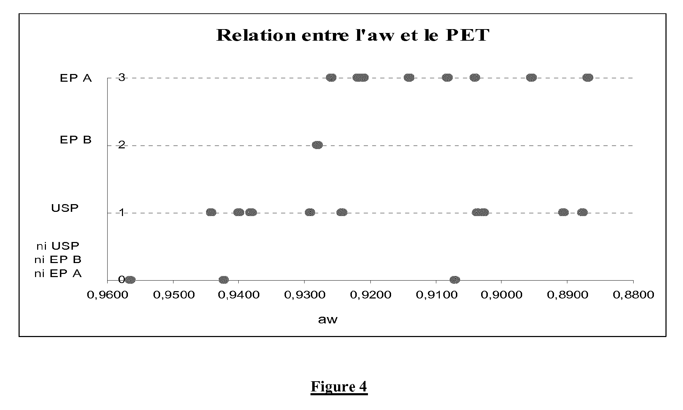

### Figure n°4 : Recherche de corrélation entre aw et le PET

Nous pouvons remarquer que les résultats obtenus par les PET ne sont pas en relation directe avec l'activité de l'eau des formules.
En effet, l'acceptation des critères A de la pharmacopée européenne qui sont les plus drastiques, ne sont pas satisfait uniquement par des formules ayants une basse a_{W}.
De plus, les a_{W} obtenues ne sont jamais plus basses que 0,8876. Cette teneur permet uniquement l'inhibition du *Pseu* et du *Coli.* Or certaines formules assurent l'inhibition de l'ensemble des germes en satisfaisant les critères A de la pharmacopée européenne ou les critères de l'USP.
Ce n'est donc pas la teneur en eau libre présente dans les formules qui assure la préservation mais bien l'action antimicrobienne des couples humectant / éthanol.

### Exemple 6 : Etude de l'effet des épaississants dans les mélanges éthanoliques

Les épaississants classiques du formulateur sont :
le Carbopol (Ultrez 10 NF) à 0,5%
le Natrosol (HHX 250) à 1,5%
et la gomme de Xathan (Xantural 180) à 2%.

Les épaississants étudiés n'ont pas tous le même pouvoir gélifiant. Cependant, ces gels analysés doivent avoir une viscosité similaire. C'est pourquoi les concentrations d'épaississant introduites dans ces derniers sont différentes.

### Effet sur les propriétés anti-microbiennes

L'action de ces différents gélifiants a été tout d'abord étudiée sur des mélanges eau / éthanol de concentration 10 et 15 % en alcool qui sont les concentrations critiques.

Le Natrosol est l'épaississant qui permet de réduire la concentration d'éthanol de 25% à 15% en satisfaisant les critères A de la pharmacopée européenne.

L'ensemble des épaississants testés permettent de réduire la concentration d'éthanol de 15 à 10% en satisfaisant les critères de l'USP.
La gomme de xanthan n'assure pas une bonne réduction du *Sta.* C'est pourquoi, 15% d'éthanol couplé à la gomme de xanthan assure seulement les critères B de la pharmacopée européenne. Le Carbopol et le Natrosol couplés à 15% d'éthanol assure la réduction des bactéries en satisfaisant les critères A de la pharmacopée européenne.

La grande différence réside dans l'élimination de l'*Aspergillus Niger.* Seul le couplage éthanol/Natrosol assure son élimination (2.4 log de réduction en 14 jours) conformément aux critères A de la pharmacopée européenne.
Leurs actions ne sont donc pas similaires. Il est possible de déterminer un ordre d'action antimicrobienne.

### Natrosol > Carbopol > Gomme de Xanthan

Il est donc plus intéressant de sélectionner le Natrosol couplé à 15% d'éthanol pour son action sur l'*Aspergillus Niger.*

L'activité de l'eau des différents gels a été mesurée.
La présence de chaque épaississant a pour conséquence de faire chuter plus ou moins l'activité de l'eau.
L'abaissement le plus important est observé avec le Natrosol lorsqu'il est introduit dans 15% d'éthanol.

### Exemple 7 : Etude de l'effet des épaississants dans les mélanges glyco-éthanoliques

Comme démontré précédemment, les épaississants ont la faculté de faire chuter l'activité de l'eau et donc d'assurer une préservation à un taux plus faible en éthanol en déplaçant la zone d'acceptabilité.
Toujours dans l'optique de réduire la quantité d'éthanol apporté à la solution, le cumul de l'action des humectants et des épaississants est à envisager.

L'épaississant choisi est la Natrosol à 1.5% et les humectants choisis sont le PG et le PEG 400.

### Propylène glycol

Deux formules ont été analysées :
Natrosol 1.5%, PG 30% : gel 1
Natrosol 1.5%, 10% Ethanol, 15% PG : gel 2

Ces formules sont testées pour déterminer s'il est possible d'éliminer complètement l'éthanol des formules (gel 1) et s'il est possible de déplacer de la zone d'acceptabilité des critères A ou B de la pharmacopée européenne.
Sans le gélifiant, ces formules ne passaient ni les critères A, ni les critères B de cette pharmacopée.

L'analyse faite sur le gel 1 démontre qu'il ne satisfait pas les critères A et B de la pharmacopée européenne. Le Natrosol n'a pas apporté pour ces concentrations une variation de la zone d'acceptation des critères de la pharmacopée européenne.

Pour le gel 2, les critères A sont satisfaits pour l'ensemble des germes à l'exception de l'*Aspergillus Niger.* La réduction observée pour celui-ci est de 1,2 log. Ceux sont donc uniquement les critères B qui sont satisfaits.

Le Natrosol apporte une certaine amélioration sur l'acceptation des critères de cette pharmacopée. La variation de l'a_{w} a une conséquence directe à certaines concentrations puisque gélifié le melange 10% d'éthanol et 15% de PG passe les critères B de la pharmacopée européenne.

### PEG 400

Deux formules ont été analysées pour les mêmes raisons que précédemment
Natrosol 1.5%, PEG 30% : gel 3
Natrosol 1.5%, 10% Ethanol, 10% PEG : gel 4

L'action du PEG est moins importante que celui du PG dans les mêmes conditions. Les gels PEG étudiés ne permettent pas de faire varier la zone d'acceptabilité des critères de la pharmacopée européenne et de l'USP.

### Conclusion sur les systèmes éthanoliques :

L'association de l'éthanol avec des glycols, en particulier le propylène glycol, est une option intéressante puisque qu'ils ont la faculté de faire chuter l'activité de l'eau. Le PG possède également une action bactéricide importante qui permet de réduire la concentration en alcool. Le rapport entre l'éthanol et la phase aqueuse assurant la préservation chute à 15%.
Le même rapport a été déterminé grâce à l'étude des gels Natrosol hydro-éthanolique.

Exemple de mélange glyco éthanolique utilisable dans une composition de type émulsion ou lotion :
15% d'éthanol, 5% de PG et 3.5% de glycérol.

Cette association de glycol a permi de mettre en évidence leur synergie car cette émulsion passe largement les critères A de la pharmacopée européenne.

### Exemple 8 : Autres additifs ayant une efficacité anti-microbienne.

Le but de cette étude est de travailler sur les familles de molécules qui pourraient avoir une action antimicrobienne grâce à des fonctions chimiques spécifiques.
Les chélatants et les antioxydants vont être analysés au sein d'une émulsion type de la formulation.
L'émulsion choisie est classique pouvant être considérée comme une émulsion typique du service de formulation.

La composition de la formule est :

| | **CONSTITUANTS** | **Pourcentage w/w** |
|---|---|---|
| **Phase grasse** | Alcool stéarylique | 4.90 |
| | EUMULGIN B2 PH | 2.10 |
| | Alcool cétylique | 1.50 |
| | Huile de paraffine | 15.00 |
| **Phase aqueuse** | Eau stérile | Aller jusqu'à 100% |

Cette formule va être la base de l'ensemble des analyses suivantes. Ainsi, les molécules qui vont être testées y seront introduites.

Cette formule a été analysée en utilisant le test d'efficacité des conservateurs (PET) afin de vérifier que cette formule ne contient aucun élément qui pourrait inhiber la prolifération microbienne.

**Tableau n°11 : Résultats de l'analyse de l'émulsion type**

| | **Réduction en log** | | | |
|---|---|---|---|---|
| | **Contrôle** | **T48h** | **T7j** | **T14j** |
| *Coli* | 6.0 | 0.1 | 0.1 | 0.1 |
| *Sta* | 6.0 | 0.2 | 0.3 | 1.7 |
| *Pseu* | 6.1 | 0 | -0.2 | -0.2 |
| *Can* | 6.0 | --- | --- | 0 |
| *Asp* | 6.0 | --- | --- | 0 |

Il est bien visible que l'émulsion choisie n'a aucune action antimicrobienne puisqu'aucune décroissance significative de germes n'est observée durant les temps de l'analyse. Cette émulsion ne satisfait aucun critère d'aucune pharmacopée. Les réductions logarithmiques sont très faibles voir inexistante pour l'ensemble des germes.

Ainsi, les éventuelles décroissances qu'il sera possible d'observer au cours des analyses suivantes seront bien dues aux effets antimicrobiens des composés testées.

Les concentrations qui ont été utilisées sont les concentrations limites supérieures tolérées dans le domaine pharmaceutique. Ainsi, si les critères ne sont pas satisfaits, il ne sera pas nécessaire de retenir le constituant comme potentiel inhibiteur de microorganismes.

### Les chélatants, l'EDTA disodium, dihydraté.

L'EDTA a été introduit dans la phase aqueuse à 0.15%. Cette concentration est la concentration maximale à utiliser dans le domaine pharmaceutique.

### Les antioxydants

Les antioxydants qui sont présenté ci-dessous, sont des antioxydants couramment utilisés en formulation.

### Antioxydants analysés

| | | |
|---|---|---|
| **Les antioxydants naturels** | L'Acide Citrique | 2 % dans la phase aqueuse |
| | L'Ascorbate de Sodium | 2% dans la phase aqueuse |
| | Le Tocophérol | 0.05% dans l'émulsion faite et refroidie car la molécule est sensible à la chaleur. |
| **Les antioxydants synthétiques** | Le BHT | 1% dans la phase grasse |
| | Le Propylgalate | 0.1% dans la phase grasse |
| | L'Ascorbyl Palmitate | 1% dans la phase aqueuse à 70°C |

### Les résultats

**Tableau n°12 : Analyse des résultats obtenus avec chacun des additifs sur chaque germe étudié**

| **Additifs** | **%** | **Moisissure s** | **Levures** | **Bactérie Gram +** | **Bactérie Gram** | **-** |
|---|---|---|---|---|---|---|
| | | *Aspergillus Niger* | *Candida Albicans* | *Staphilococcus aureus* | *Pseudomona s aeruginosa* | *Eschérichia Coli* |
| Emulsion blanche | | - | - | - | | |
| EDTA | 0.15 | - | - | - | ++ | |
| Acide citrique | 2 | - | ++ | ++ | ++ | + |
| Ascobate de sodium | 2 | - | - | ++ | | + |
| Tocophérol | 0.05 | - | - | - | | |
| BHT | 1 | - | - | - | | |
| Propylgalate | 0.1 | - | - | - | ++ | |
| Ascorbyl palmitate | 1 | - | - | ++ | ++ | + |
| **Total** | | **-** | **++** | **++** | **++** | **+** |

| | | | | | | |
|---|---|---|---|---|---|---|
| *Légende :* - *aucun critère n'est satisfait, ni de la pharmacopée européenne, ni de l'USP.* *+ pour le coli : les critères de l'USP sont satisfaits* ++ *: les critères A de la pharmacopée européenne sont satisfaits* | | | | | | |

L'étude des différentes actions de ces additifs révèle une grande diversité de leur comportement envers les microorganismes.

Il est possible de distinguer 3 comportements bien distincts.
✔ Le tocophérol et le BHT n'ont pas une efficacité significative sur l'ensemble des germes. Leur utilisation ne sera donc pas retenue pour assurer une préservation antimicrobienne.
✔ L'EDTA et le Propylgalate inhibent uniquement le *Pseudomonas aeruginosa* en satisfaisant les critères A de la pharmacopée européenne.

L'Ascorbate de Sodium a une très bonne action sur le *Staphilococcus aureus* en satisfaisant les critères A de la pharmacopée européenne et l'*Eschérichia Coli* en satisfaisant les critères de l'USP.
✔ L'Acide Citrique et l'Ascorbyl Palmitate ont un spectre d'efficacité plus large. En effet, tout deux sont bactéricides. L'action de l'Acide Citrique est plus importante car il inhibe en plus des bactéries, le *Candida Albicans* grâce à un pH très bas (pH = 2.04).

### Conclusion

Les résultats montrent qu'il n'est pas possible d'obtenir un spectre d'action complet inhibant la totalité des germes.
*L'Aspergillus Niger* est bloquant. Aucun des additifs étudiés n'a permis une inhibition même minime.

L'élimination des moisissures est le problème récurant pour chacune des voies d'autoprotection étudiées. Seul l'éthanol à forte concentration a permis d'inhiber son développement.
Il est donc important de déterminer un système de préservation qui assure l'inhibition de l'ensemble des germes et en particulier de l'*Aspergillus Niger.*

### Exemple 9 : Utilisation partielle d'un système de conservateur classique.

Afin d'assurer la total préservation d'une matrice, il est important que le système conservateur assure l'inhibition des bactéries et des fongiques.

Classiquement, une combinaison de conservateurs antimicrobiens sont utilisés, plusieurs parabènes et du phénoxyéthanol.
Les concentrations de ces conservateurs sont réglementées, le mélange des parabènes ne doit pas excéder les 0.8% de la formule et la concentration du phénoxyéthanol est limitée à 1%. L'ensemble du système conservateur classique représente donc 1.8% de la masse de la formule.
L'étude précédente a mis en avant de nombreuses voies d'étude pouvant remplacer partiellement le système conservateur. Ainsi, il a été mis en avant des additifs tels que des antioxydants ou des humectants qui pouvaient assurer l'action bactéricide du système conservateur.
L'utilisation de conservateur est donc limitée à leur action fongicide.

Pour cela, il a été décidé de sélectionner l'Ascorbyl Palmitate qui assure l'action bactéricide. L'acide citrique n'a pas été choisi car l'influence du pH est un paramètre qui constituerait une étude beaucoup plus importante. Les résultats obtenus ne seraient pas significative car deux critères entrent en jeux, le pH et l'action de la molécule elle-même.
Le système fongicide peut être obtenu uniquement par le propylparaben qui a une action plus importante sur ces germes que le méthyl et éthylparabens. En effet, 250ppm de propylparaben permet d'inhiber l'aspergillus Niger et le *Candida Albicans* alors qu'il faudrait 1000ppm de méthylparaben pour obtenir le même résultat. [9]
Afin de réduire au maximum l'apport de conservateurs antimicrobiens, plusieurs concentrations de propylparaben ont été testées.
Une gamme de concentration allant de 0.4% (pourcentage maximal autoriser par la réglementation) à 0.1% par pas de 0.1% a été analysée.

Compositions des émulsions analysées

| **N°** | **CONSTITUANTS** | % | % | % | % |
|---|---|---|---|---|---|
| Phase Organique | Alcool stéarylique | 4,9 | 4,9 | 4,9 | 4,9 |
| | EUMULGIN B2 PH | 2,1 | 2,1 | 2,1 | 2,1 |
| | Alcool cetylique | 1,5 | 1,5 | 1,5 | 1,5 |
| | Huile de paraffine | 15 | 15 | 15 | 15 |
| Phase Aqueuse | Eau stérile | 75,1 | 75,2 | 75,3 | 75,4 |
| Antioxydant | ascorbyl palmitate | 1 | 1 | 1 | 1 |
| Conservateurs | **propyl paraben** | **0,4** | **0,3** | **0,2** | **0,1** |

### Les résultats du PET

**Tableau n°13 : Résultats de l'efficacité du système Ascorbyl palmitate / propylparaben**

| Emulsion à 0,4% de propylparaben | | | | |
|---|---|---|---|---|
| | | **Réduction en log** | | |
| | **Contrôle** | **T48h** | **T7j** | **T14j** |
| *Coli* | 5,9 | >3,9 | 4,8 | 5,9 |
| *Sta* | 5,9 | >3,9 | 4,2 | 4,8 |
| *Pseu* | 5,9 | >3,9 | 4,3 | 4,5 |
| *Can* | 5,9 | --- | --- | >4,0 |
| *Asp* | 5,9 | --- | --- | 3,3 |

| Emulsion à 0,3% de propylparaben | | | | |
|---|---|---|---|---|
| | **Réduction en log** | | | |
| | **Contrôle** | **T48h** | **T7j** | **T14j** |
| *Coli* | 5,9 | >3,9 | 4,5 | 5,9 |
| *Sta* | 5,9 | >3,9 | 4,7 | 4,7 |
| *Pseu* | 5,9 | >3,9 | 4,8 | 4,9 |
| *Can* | 5,9 | --- | --- | >4,0 |
| *Asp* | 5,9 | --- | --- | 3,0 |

| Emulsion à 0,2% de propylparaben | | | | |
|---|---|---|---|---|
| | **Réduction en log** | | | |
| | **Contrôle** | **T48h** | **T7j** | **T14j** |
| *Coli* | 5,9 | >3,9 | 5,9 | 5,9 |
| *Sta* | 5,9 | >3,9 | 4,8 | 5,9 |
| *Pseu* | 5,9 | >3,9 | 4,8 | 5,9 |
| *Can* | 5,9 | --- | --- | >4,0 |
| *Asp* | 5,9 | --- | --- | 2,0 |

| Emulsion à 0,1% de propylparaben | | | | |
|---|---|---|---|---|
| | **Réduction en log** | | | |
| | **Contrôle** | **T48h** | **T7j** | **T14j** |
| *Coli* | 5,9 | >3,9 | 3,7 | 4,6 |
| *Sta* | 5,9 | >3,9 | 3,7 | 4,2 |
| *Pseu* | 5,9 | >3,9 | 3,8 | 4,2 |
| *Can* | 5,9 | --- | --- | 4,8 |
| *Asp* | 5,9 | --- | --- | 2,0 |

L'ensemble des formules étudiées par PET passe tous les critères des pharmacopées européenne et américaine. Cependant, pour les formules contenant 0,2% et 0,1% de propylparaben, la réduction de *l'Asp* observée est juste égale à 2,0. Pour plus de sécurité dans les résultats, la formule sélectionnée est celle contenant 0,3% de propylparaben et 1% d'ascorbyl palmitate.

Le pourcentage introduit de conservateur antimicrobien dans cette émulsion est de 0,3% contre 1,8% dans des émulsions classiques. La réduction de parabènes et de conservateurs en règle générale a donc été obtenue grâce à l'utilisation d'1% d'ascorbyl palmitate.

### Exemple 10 : Combinaison des additifs étudiés dans les exemples précédents

Afin d'éliminer totalement le système conservateur classique d'une composition, le mélange suivant est testé :
- Un antioxydant, l'ascorbyl palmitate à 1% en masse
- Un humectant, le propylène glycol à 5% en masse
- Un pourcentage d'eau plus faible que précédemment, 50% en masse
- Un rapport éthanol / phase aqueuse allant de 10 à 15 %.

Compositions des formules analysées

| **N°** | **CONSTITUANTS** | **%** | **%** |
|---|---|---|---|
| Phase Organique | Alcool Stéarylique | 4,9 | 4,9 |
| | EUMULGIN B2 PH | 4 | 4 |
| | Alcool Cétylique | 1,5 | 1,5 |
| | Huile de paraffine | 39,6 | 39,6 |
| Phase Aqueuse | Eau stérile | 38,13 | 39,9 |
| | **éthanol** | **5,87** | **4,1** |
| | PG | 5 | 5 |
| Antioxydant | Ascorbyl Palmitate | 1 | 1 |

### Les résultats du PET

**Tableau n°14 : Résultats des émulsions n° 1 et n°2**

| Emulsion n°1 R _{éthanol/phase aqueuse} = 15 | | | | |
|---|---|---|---|---|
| | **Réduction en log** | | | |
| | **Contrôle** | **T48h** | **T7j T14j** | |
| *Coli* | 5,9 | >3,9 | 5,9 | 5,9 |
| *Sta* | 5,9 | >3,9 | 5,9 | 5,9 |
| *Pseu* | 5,9 | >3,9 | 5,9 | 5,9 |
| *Can* | 5,9 | --- | --- | >4,0 |
| *Asp* | 5,9 | --- | --- | >3,9 |

| Emulsion n°2 R _{éthanol/phase aqueuse} = 10 | | | | |
|---|---|---|---|---|
| | **Réduction en log** | | | |
| | **Contrôle** | **T48h** | **T7j** | **T14j** |
| *Coli* | 5,9 | >3,9 | 5,9 | 5,9 |
| *Sta* | 5,9 | >3,9 | 5,9 | 5,9 |
| *Pseu* | 5,9 | >3,9 | 5,9 | 5,9 |
| *Can* | 5,9 | --- | --- | >4,0 |
| *Asp* | 5,9 | --- | --- | >3,9 |

L'ensemble des critères des pharmacopées est satisfait très largement. En effet, les réductions observées sont très élevées. La réduction de log de *l'Aspergillus Niger* au temps 14 jours est supérieure à 3,9 pour les deux formules. L'émulsion n°2 contenant le moins d'éthanol peut être sélectionnée car son action est aussi bonne que l'émulsion n°1.
L'activité de l'eau ne joue pas ici un rôle très important car à cette valeur, seulement le Coli et le Pseu sont inhibés.
L'action antimicrobienne observée est due à l'effet synergique de l'association de l'ascorbyl palmitate, de l'éthanol et du PG.

## Revendications

1. Mélange d'excipient comprenant :
- de l'éthanol,
- de l'ascorbyl palmitate à une concentration de 1% en masse,
- du propylène glycol à 5% en masse,
- un pourcentage en eau inférieur à 50% en masse, et
- un rapport éthanol/phase aqueuse allant de 10 à 15 %.

2. Mélange d'excipient selon la revendication 1, **caractérisé en ce qu'**il comprend :
- entre 5 et 25% d'éthanol.

3. Mélange d'excipient selon la revendication 1 ou 2, caractérisé ce qu'il comprend un épaississant présent à une concentration d'au moins 0,5 % en masse.

4. Utilisation d'un mélange d'excipient selon l'une des revendications 1 à 3, en tant que conservateur au sein d'une composition pharmaceutique topique.

5. Utilisation d'un mélange d'excipient selon la revendications 4 au sein d'une composition pharmaceutique topique contenant de 0 à 1,8% en masse de conservateurs choisis parmi les générateurs de formaldéhylde, tel l'imidazolidinylurée, ou la diazolidinylurée, les dérivés thiazinique tel le Kathon CG, les dérivés chlorés, tel la chlorhexidine, les dérivés phénoliques, tel les parabens, les dérivés alcooliques, tel le bronopol, le phénoxyéthanol, le chloroxylenol, les dérivés acides, tel l'acide sorbique, l'acide benzoïque et/ou le chlorure de benzalkonium.

6. Utilisation d'un mélange d'excipient selon la revendication 5, **caractérisée en ce que** la composition pharmaceutique contient moins de 0,8% de parabens.

7. Utilisation d'un mélange d'excipient selon la revendication 6, **caractérisée en ce que** la composition pharmaceutique contient moins de 0,4% de propylparabens.

8. Utilisation d'un mélange d'excipient selon l'une des revendications 1 à 3, pour éviter la prolifération des microorganismes au sein d'une composition pharmaceutique topique.

## Patentansprüche

1. Trägergemisch, umfassend:
- Ethanol,
- Ascorbylpalmitat mit einer Konzentration von 1 Gew.-%,
- Propylenglycol mit 5 Gew.-%,
- ein Prozentgehalt an Wasser unter 50 Gew.-%, und
- ein Verhältnis Ethanol / wässrige Phase von 10 bis 15%.

2. Trägergemisch gemäß Anspruch 1, **dadurch gekennzeichnet, dass** es umfasst:
- zwischen 5 und 25% Ethanol.

3. Trägergemisch gemäß Anspruch 1 oder 2, **dadurch gekennzeichnet, dass** es ein Verdickungsmittel umfasst, das in einer Konzentration von weniger als 0,5 Gew.-% vorliegt.

4. Verwendung eines Trägergemisches gemäß einem der Ansprüche 1 bis 3 als Konservierungsstoff in einer topischen pharmazeutischen Zusammensetzung.

5. Verwendung eines Trägergemisches gemäß Anspruch 4 in einer topischen pharmazeutischen Zusammensetzung, enthaltend 0 bis 1,8 Gew.-% Konservierungsstoffe, ausgewählt aus Formaldehydabspaltern, wie Imidazolidinylharnstoff oder Diazolidinylharnstoff, Thiazinderivaten, wie Kathon CG, chlorierten Derivaten, wie Chlorhexidin, Phenolderivaten, wie Parabenen, Alkoholderivaten, wie Bronopol, Phenoxyethanol, Chlorxylenol, Säurederivaten, wie Sorbinsäure, Benzoesäure und/oder Benzalkoniumchlorid.

6. Verwendung eines Trägergemisches gemäß Anspruch 5, **dadurch gekennzeichnet, dass** die pharmazeutische Zusammensetzung weniger als 0,8% Parabene enthält.

7. Verwendung eines Trägergemisches gemäß Anspruch 6, **dadurch gekennzeichnet, dass** die pharmazeutische Zusammensetzung weniger als 0,4% Propylparabene enthält.

8. Verwendung eines Trägergemisches gemäß einem der Ansprüche 1 bis 3, um die Vermehrung von Mikroorganismen in einer topischen pharmazeutischen Zusammensetzung zu verhindern.

## Claims

1. A mixture of excipients comprising:
- ethanol,
- ascorbyl palmitate at a concentration of 1% by mass,
- propylene glycol at a concentration of 5% by mass,
- a percentage of water inferior to 50% by mass, and
- an ethanol/aqueous phase ratio ranging from 10% to 15%.

2. The mixture of excipients according to claim 1, wherein it comprises:
- between 5% and 25% of ethanol.

3. The mixture of excipients according to claim 1 or 2, wherein it comprises a thickener at a concentration of at least 0.5% by mass.

4. Use of a mixture of excipients according to any one of claims 1 to 3, as a conservative within a topical pharmaceutical composition.

5. Use of a mixture of excipients according to claim 4 within a topical pharmaceutical composition containing containing 0 to 1.8% by mass of conservatives selected from the group consisting of formaldehyde-generating agents, such as imidazolidinylurea, or diazolidinylurea, thiazine compounds, chlorinated compounds, such as chlorhexidine, phenolic compounds, such as parabens, alcoholic compounds, such as bronopol, phenoxyethanol, chloroxylenol, acid compounds, such as ascorbic acid, benzoic acid, and/or benzalkonium chloride.

6. Use of a mixture of excipients according to claim 5, wherein said pharmaceutical composition contains less than 0.8% of parabens.

7. Use of a mixture of excipients according to claim 6, wherein said composition contains less than 0.4% of propylparaben.

8. Use of a mixture of excipients according to any one of claims 1-3, for avoiding the microorganism proliferation within a topical pharmaceutical composition.
